Europäisches Patentamt

European Patent Office

Office européen des brevets

(19)

(11) Veröffentlichungsnummer : **0 486 632 B1**

(12) **EUROPÄISCHE PATENTSCHRIFT**

(45) Veröffentlichungstag der Patentschrift :
27.07.94 Patentblatt 94/30

(51) Int. Cl.$^5$ : **A61K 7/42,** A61K 31/505,
A61K 31/455

(21) Anmeldenummer : 91909455.7

(22) Anmeldetag : 24.05.91

(86) Internationale Anmeldenummer :
PCT/DE91/00433

(87) Internationale Veröffentlichungsnummer :
WO 91/19478 26.12.91 Gazette 91/29

(54) **LICHTSCHUTZ.**

(30) Priorität : 13.06.90 DE 4018964

(43) Veröffentlichungstag der Anmeldung :
27.05.92 Patentblatt 92/22

(45) Bekanntmachung des Hinweises auf die
Patenterteilung :
27.07.94 Patentblatt 94/30

(84) Benannte Vertragsstaaten :
AT BE CH DE DK ES FR GB GR IT LI LU NL SE

(56) Entgegenhaltungen :
EP-A- 0 330 583
DE-A- 2 242 553
FR-A- 2 096 712

(73) Patentinhaber : WEBER, Gerhard, Prof.Dr.
Lempenmühle
D-96172 Mühlhausen (DE)

(72) Erfinder : WEBER, Gerhard, Prof.Dr.
Lempenmühle
D-96172 Mühlhausen (DE)

(74) Vertreter : Hübner, Gerd, Dipl.-Phys. et al
Rau, Schneck & Hübner
Patentanwälte
Königstrasse 2
D-90402 Nürnberg (DE)

EP 0 486 632 B1

**Beschreibung**

Die Erfindung betrifft ein pharmazeutisches Mittel für die systemische Prophylaxe von Hautschäden, die durch Licht, insbesondere Sonnenlicht, hervorgerufen werden, sowie die Verwendung von Nicotinsäureamid und Folsäure zur Herstellung dieses oral oder parenteral verabreichbaren pharmazeutischen Mittels.

Lichtschutzmittel, insbesondere Sonnenschutzmittel, für den Schutz der menschlichen Haut gegenüber schädigenden Einflüssen der direkten oder indirekten Strahlung (Licht, insbesondere Sonnenlicht) sind bereits bekannt. Im Handel erhältlich sind in erster Linie Sonnenöle, Sonnenmilche, Sonnenemulsionen, Sonnencremes, Sonnengelees, Sonnenlotionen, Sonnensprays und Sonnensprayemulsionen. Ihre Wirksamkeit ist von der Art der verwendeten Lichtfilter und deren Konzentration abhängig.

Manchen Sonnenschutzmitteln werden zusätzliche Agentien, wie Dihydroxyaceton, Carotin oder Walnuß-Schalenextrakte zugesetzt, um eine künstliche Bräunung der Hornschicht der Haut, die aus ästhetischen Gründen erwünscht ist, zu bewirken.

Im allgemeinen führt die Einwirkung von Licht, insbesondere von Sonnenlicht, auf das menschliche Hautorgan zu einer physiologischen, nicht-pathologischen Reaktion, dem Erythema solare bzw. solaris, d.h. einer Rötung der Haut durch die einwirkende Strahlung. Bei einer Bestrahlung über das normale Maß hinaus wird eine pathologische Reaktion, die Dermatitis solaris, d.h. eine Entzündung der Haut durch Licht, insbesondere Sonnenlicht, hervorgerufen. Die Übergänge von Erythema solaris zu Dermatitis solaris sind fließend und individuell unterschiedlich.

Sie hängen einerseits ab von der Intensität des einwirkenden Lichtes (Sonnenlichtes), andererseits vom natürlichen Hautschutz gegen das Licht (Sonnenlicht) und damit von der individuellen Lichtempfindlichkeit, die normal oder gesteigert und damit pathologisch sein kann. Sonnenlicht ist in seiner Zusammensetzung weitgehend konstant, wobei UV-Licht A, B, C, sichtbares Licht und Infrarotlicht auf die menschliche Haut einwirken, wobei das UV-B für die Hautrötung und das UV-A für die Hautpigmentierung verantwortlich gemacht werden. Die Lichtintensitäten können variabel sein, was auch vom Lichteinfallswinkel abhängt.

Seitens des menschlichen Hautorgans wird der Sonnenschutz bewirkt (1) durch die Dicke der Hornschicht der Haut (sogenannte Miescher'sche Lichtschwiele) und (2) durch die Dichte des in der Haut enthaltenen Melanins, des körpereigenen Hautpigments. Die quantitative Zunahme beider Faktoren ermöglicht einen Lichtschutz und vice versa.

Bisher galt, daß die Haut durch Auftragen von Lichtschutzfaktoren bedingt gegen die Einwirkung des Lichtes (Sonnenlichtes) geschützt werden kann. Andererseits wird durch Auftragen oder perorale Einnahme von photosensibilisierenden Substanzen, z.B. Fukomarinen, die Lichtempfindlichkeit des Hautorgans erhöht, ein Vorgang, der u.a. als Lichtsensibilisierung bezeichnet wird. Unabhängig davon gibt es auch sogenannte Lichtdermatosen, die entweder durch körpereigene lichtsensibilisierende Substanzen wie Porphyrine entstehen oder unbekannter Ursache sind.

Es ist bekannt, daß krankhafte Lichtreaktionen im Sinne der Lichtdermatosen beispielsweise durch Cortison, Chloroquin oder β-Carotine behandelt werden können, allerdings mit fraglichem Erfolg. Eine dieser sehr unterschiedlichen Lichterkrankungen ist die polymorphe Lichtdermatose, auch als summer eruption (summer prurigo) bezeichnet, für die es derzeit keine Therapie gibt. Auch eine Methode zur systemischen Prophylaxe der polymorphen Lichtdermatose oder der gesteigerten Lichtempfindlichkeit ist bisher nicht bekannt.

Sonnenschutzmittel sind Lichtschutzmittel für den Schutz der menschlichen Haut gegenüber schädigenden Einflüssen der direkten und indirekten Bestrahlung mit Licht, insbesondere Sonnenlicht.

Die für die oft erwünschte Hautbräunung verantwortliche UV-Strahlung der Sonne unterteilt man in UV-C (Wellenlängen 200 - 280 nm), UV-B (280 - 315 nm) und UV-A (315 - 400 nm). Die Intensität der wirksamen Sonnenstrahlung ist von geographischen, klimatischen (Schnee-Reflexion) und anderen Faktoren und gegebenenfalls auch von Luftverunreinigungen abhängig. Das kurzwellige UV-C tritt am Erdboden nicht in Erscheinung, da diese Strahlung in der Atmosphäre durch das dort vorhandene Ozon absorbiert wird.

Die Pigmentierung der normalen Haut unter dem Einfluß der Sonnenstrahlung, d.h. die Bildung von Melanin, wird durch UV-B und UV-A in unterschiedlicher Weise bewirkt. Bestrahlung mit UV-A-Strahlen hat das direkte Dunkelwerden der in der Epidermis bereits vorhandenen Melaninkörper zur Folge. Das UV-B bewirkt die indirekte Pigmentierung. Ehe jedoch das (schützende) Pigment gebildet ist, unterliegt die Haut der Einwirkung der ungefilterten Strahlung, die - je nach Expositionsdauer - zur Bildung von Hautrötungen (Erythemen), Hautentzündungen (Sonnenbrand) und sogar Blasenbildungen führen kann. Die mit derartigen Hautläsionen verbundenen Belastungen des Organismus können Kopfweh, Mattigkeit, Fieber sowie Herz- und Kreislaufstörungen verursachen.

Auf die Haut aufgebrachte Sonnenschutzmittel haben den Zweck, die sonnenbranderzeugenden Strahlungsteile zurückzuhalten und die hautbräunenden Lichtwellen unverändert passieren zu lassen. Als UV-Absorber oder Lichtfilter kommen Benzophenonderivate, Hydroxynaphthochinone, Phenylbenzoxazole und Phenylbenzimidazole, Digalloyltrioleat, Aminobenzoesäureester, Salicylsäureester, alicyclische Dienone, Zimtsäureester und Benzalazin in Frage. Auch aromatische Harnstoffderivate und bestimmte Sulfonamide, Cumarinderivate und Phenylglyoxylsäurederivate sind bereits als Sonnenschutzmittel vorgeschlagen worden. Sonnenschutzmittel auf natürlicher Basis sind z.B. Nerz-, Avocado-, Mandel-, Sesam-, Erdnuß-, Oliven-, Safflor-, Kokosöl und andere Öle.

Nachteilig an den bisher bekannten Lichtschutz- bzw. Sonnenschutzmitteln ist die Tatsache, daß jede zu schützende Körperstelle durch örtlichen Auftrag auf die Haut mehr oder weniger stark gegen Sonneneinstrahlung geschützt werden muß, ohne daß eine sichere Gewähr dafür gegeben ist, daß der Schutz sich voll entfaltet und die Person nicht dennoch einen Sonnenbrand erleidet. Ungeschützte Körperstellen, z.B. die Kopfhaut, die ungewollt der Sonnenstrahlung ausgesetzt sind, sind meistens besonders stark von Sonnenbrand befallen. Ferner wird durch Abrieb, Waschen, Baden und Körperschweiß der größte Teil des topisch aufgebrachten Sonnenschutzmittels (Öl, Creme) entfernt, so daß häufig ein erneuter Auftrag notwendig wird, was lästig, gegebenenfalls auch unzuverlässig und vielfach auch nur unvollständig ist, da es nur unter experimentellen Bedingungen möglich ist, den Lichtfilter in gleicher Schichtdicke aufzutragen. Außerdem gibt es kaum ein Mittel, das gegen das gesamte Spektrum des Sonnenlichtes voll wirksam ist.

Aus DE-A-22 42 533 ist ein kosmetisches Mittel bekannt, das für den topischen Auftrag auf die Haut und auf die Haare geeignet ist und deodorierende Eigenschaften besitzt. Es enthält Folsäure in Kombination mit physiologisch verträglichen Kationen als aktives Agens, Wasser als Träger und Nicotinsäureamid sowohl als Lösungsmittel als auch als Deodorierungsmittel. Hinweise darauf, daß dieses kosmetische Mittel für die systemische Prophylaxe von durch Licht, insbesondere Sonnenlicht, hervorgerufenen Hautschäden geeignet ist, finden sich darin jedoch nicht.

Aus EP-A-0 330 583 ist ein kosmetisches Mittel zur Verbesserung des ästhetischen Aussehens der Haut bekannt, das ein geeignetes kosmetisches Vehiculum sowie ein Gemisch aus den Vitaminen E, A, B2, B5 und H enthält. Außerdem kann es noch die Vitamine F, D, B6 und Folsäure enthalten. Nicotinsäureamid ist in diesem bekannten kosmetischen Mittel nicht enthalten.

Aus FR-A-2 096 712 ist eine topisch verabreichbare Zusammensetzung bekannt, die Riboflavin und/oder Nicotinsäureamid zusammen mit einem Hydrocortisonderivat enthält. Diese Zusammensetzung ist nach den Angaben in dieser Druckschrift für die Behandlung von Pellagra bekannt. Folsäure enthält dieses bekannte Antipellagra-Mittel jedoch nicht.

Aufgabe der Erfindung war es, ein Lichtschutzmittel zu finden, das eine wirksame systemische Prophylaxe gegen Hautschäden ermöglicht, die durch Licht, insbesondere Sonnenlicht, hervorgerufen werden, und das alle Körperteile mit ihrem Hautorgan in den Lichtschutz einbezieht und einen Langzeit-Lichtschutz ergibt.

Es wurde nun gefunden, daß diese Aufgabe erfindungsgemäß dadurch gelöst werden kann, daß Nicotinsäureamid in Kombination mit Folsäure in einem spezifischen Mengenverhältnis als aktiver Wirkstoff eingesetzt wird.

Gegenstand der Erfindung ist ein pharmazeutisches Mittel für die systemische Prophylaxe von Hautschäden, die durch Licht, insbesondere Sonnenlicht, hervorgerufen werden, das dadurch gekennzeichnet ist, daß es als Wirkstoff Nicotinsäureamid in Kombination mit Folsäure im Gewichtsverhältnis 10:1 bis 60:1, vorzugsweise 30:1 bis 50:1, besonders bevorzugt 40:1, sowie übliche Trägerstoffe oder Vehicula enthält.

Das erfindungsgemäße pharmazeutische Mittel kann oral oder parenteral verabreicht werden und bietet einen systemischen Schutz, der alle Teile der Haut erfaßt und wirksam gegen die krankheitsauslösende Wirkung von Licht, insbesondere Sonnenlicht, über einen Zeitraum von mehr als 12 Stunden schützt. Das erfindungsgemäße pharmazeutische Mittel führt zu einem Schutz der gesamten Haut vor physiologischen nichtphathologischen und pathologischen Lichtreaktionen.

Zwar bewirkt auch Nicotinsäureamid allein bereits einen gewissen Lichtschutz, wobei Tagesmengen bei einem erwachsenen Menschen bis zu 3000 mg erforderlich sind, und auch Folsäure allein entfaltet eine gewisse Lichtschutzwirkung in Tagesmengen von 10 bis 30 mg. Es hat sich jedoch überraschenderweise gezeigt, daß eine Kombination von Nicotinsäureamid und Folsäure in einem Gewichtsverhältnis von 10:1 bis 60:1, vorzugsweise von 30:1 bis 50:1, insbesondere von 40:1, eine synergistische Wirkung insofern hat, als die erforderliche Menge, in der die beiden Wirkstoffe in Kombination verabreicht werden müssen, deutlich geringer ist als bei Anwendung jeder der Einzelverbindungen.

Die Lichtschutzwirkung von Nicotinsäureamid und/oder Folsäure bei systemischer Applikation ist insofern überraschend und auch für den Fachmann auf diesem Gebiet nicht vorhersehbar, als diese Vitamine einerseits mit den üblichen Vitaminpräparaten und andererseits als Bestandteil von Medikamenten (Nicotinsäureamid) bereits seit langem bekannt sind, ohne daß jemals die systemische Lichtschutzwirkung dieser Kombination von Verbindungen erprobt worden ist.

Das erfindungsgemäße pharmazeutische Mittel ist oral und parenteral verabreichbar.

Gegenstand der Erfindung ist ferner die Verwendung von Nicotinsäureamid und Folsäure zur Herstellung eines oral oder parenteral verabreichbaren pharmazeutischen Mittels für die systemische Prophylaxe von Hautschäden, die durch Licht, insbesondere Sonnenlicht, hervorgerufen werden.

Die Erfindung wird nachstehend unter Bezugnahme auf bevorzugte Ausführungsbeispiele näher erläutert.

Zur Herstellung geeigneter Präparate können die Wirkstoffe in entsprechender Dosierung den üblichen, oral oder parenteral zu verabreichenden Formulierungen einverleibt werden. Gängige Formulierungen sind Tabletten, Kapseln, Drageen, Säfte, Injektionslösungen oder -suspensionen, Sirupe, Inhalationsmittel und dgl.

Die für die einzelnen Formulierungen verwendbaren Vehicula oder Träger sind die gleichen, wie sie allgemein aus der Galenik bekannt sind.

Das erfindungsgemäße pharmazeutische Lichtschutzmittel kann selbstverständlich auch als Ergänzung zu den üblichen, topisch auftragbaren Sonnenschutzmitteln (Sonnenbräunungsölen, -cremes, -lotionen und -salben) eingesetzt werden.

Die Erfindung wird durch die folgenden Herstellungsbeispiele und eine Reihe von Anwendungsbeispielen näher erläutert. Das Herstellungsbeispiel 2 stellt ein oral verabreichbares erfindungsgemäßes pharmazeutisches Mittel dar, das in den Anwendungsbeispielen eingesetzt wurde.

Herstellungsbeispiel 1 (Vergleich)

Nicotinsäureamid wurde zusammen mit üblichen Tablettiermassen kompoundiert und tablettiert. Die Einzeltablette erhielt 200 mg Nicotinsäureamid.

Durch Einnahme dieser Tabletten vor Sonnenexposition trat die polymorphe Lichtdermatose bei mehreren so behandelten Personen abgeschwächt auf. Jedoch konnte selbst bei hoher Dosierung eine Manifestation nicht verhindert werden.

Herstellungsbeispiel 2

Analog Beispiel 1 wurden Nicotinsäureamid und Folsäure mit einem Gewichtsverhältnis von 40:1 (Nicotinsäureamid/Folsäure) zu Tabletten formuliert, die eine Zusammensetzung mit 200 mg Nicotinsäureamid und 5 mg Folsäure aufwiesen.

Anwendungsbeispiel 1

Ein 6-jähriger Jünge litt in jedem Frühjahr bereits nach der ersten Sonnenexposition am Auftreten einer polymorphen Lichdermatose nach Art der Summer eruption. Alle bekannten therapeutischen Möglichkeiten waren ausgeschöpft, ohne daß eine Verhinderung dieser Eruption jemals möglich gewesen wäre.

Die üblichen Behandlungen, die auch heute noch zur Linderung dieser polymorphen Lichtdermatose angewandt werden, zeigten keinen therapeutischen Effekt. So waren Corticosteroide lokal und systemisch verabreicht worden. Lichtschutzmittel jeglicher Art ebenso wie die vorgezogene Bräunung in dem Solarium erwiesen sich als ineffektiv.

Die Verabreichung des oralen Lichtschutzmittels nach Beispiel 2 führte bei dem Jüngen dazu, daß zunächst die Reexposition in südlicher Sonne ohne jegliche Hauterscheinungen im Sinne der Lichtdermatose erfolgen konnte und daß in den folgenden Jahren auch die primäre Exposition ohne Lichtunverträglichkeit möglich war.

Anwendungsbeispiel 2

Weitere Patienten, die an einer Lichtdermatose im Sinne der Summer eruption litten, wurden analog behandelt.

Der Erfolg war gleichbleibend, wobei sich eine deutliche Abhängigkeit von der Medikation insofern zeigte, als eine zu spät begonnene Behandlung mit schwachen Hauterscheinungen, eine rechtzeitig begonnene prophylaktische Behandlung ohne die pathologischen Hauterscheinungen nach Sonnenexposition verlief. Auch der quälende Juckreiz, der bekanntlich durch keine medikamentöse Therapie zu lindern ist, fehlte oder war dosisabhängig drastisch reduziert.

Patienten, die auf diese Weise geschützt werden konnten und erscheinungsfrei bei der Sonnenexposition blieben, beobachteten aber auch, daß die Unterlassung dieser Therapie bei einer erneuten Exposition zu Rückfällen führte.

Auch gaben die Patienten an, keine oder nur noch eine milde Sonnenrötung der Haut nach Sonnenexposition beobachtet zu haben, ohne daß der Bräunungseffekt der Sonnenbestrahlung vermindert wurde. Die erfindungsgemäße Kombination wurde von den Patienten mit gleichbleibendem Erfolg bei Reisen in Sonnenländer angewendet.

Es ist einfacher, beginnend 3 oder 4 Tage vor Antritt einer Urlaubsreise in die Sonne die Vitaminkombination erfindungsgemäß einzunehmen, als mit mehr oder minder wirksamen Lichtschutzmitteln das Hautorgan vor der ersten Sonnenexposition und damit meist unzureichend zu behandeln.

| Person Nr. | | Hautempfind-lichkeit | Sonnenbelastung | Befunde |
|---|---|---|---|---|
| 1 | m | immer Sb | Dom. Rep. 2 Wo. | o.Sb. |
| 2 | f | S.e. | Gardasee 2 Wo | o.Sb., o.S.e. |
| 3 | f | S.e. 5J. | Türkei 2 Wo | o.S.e. |
| 4 | f | S.e.u.Streß 10J. | Griechenl. 3 Wo | o.S.e. |
| 5 | m | Sb immer | Italien 2 Wo | |
| 6 | f | Sb immer | Italien 2 Wo | |
| 7 | f | S.e. - 10J. | Italien 2 Wo | o.S.e. |
| 8 | f | S.e. - 7J. | Teneriffa 2 Wo | o.S.e. |
| 9 | f | S.e. - 5J. | Italien 3 Wo | o.S.e. |
| 10 | f | S.e. immer | | |
| 11 | f | S.e. immer | Italien 2 Wo | o.Sb, o.S.e. |
| 12 | f | S.e. 5J. | Türkei 2 Wo | o.Sb, o.S.e. |
| 13 | f | S.e. | 5x4 Wo exponiert | o.S.e. |
| 14 | f | immer Sb | "Urlaub" 2 Wo | o.S.e. |
| 15 | f | immer Sb | Afrika 4 Wo | o.Sb |
| 16 | f | immer Sb | Griechenl. 2 Wo | o.Sb |
| 17 | f | immer Sb | Griechenl. 2 Wo | o.Sb |
| 18 | f | immer Sb | Türkei 2 Wo | o.Sb |
| 19 | f | immer Sb | "Berge" 2 Wo | o.Sb |
| 20 | m | immer Sb | "Meer" 2 Wo | o.Sb |
| 21 | f | immer Sb | Gletscher 10 T. | o.Sb |
| 22 | f | immer Sb | Italien 3 Wo | o.Sb |
| 23 | m | S.e. Sb | Ski-Url. 12 T. | o.Sb |
| 24 | m | S.e. Sb | See.Url. 2 Wo | o.S.e. |
| 25 | f | S.e. Sb | "Urlaub" 3 Wo | o.S.e. |
| 26 | f | S.e. Sb | Italien 3 Wo | o.S.e. |
| 27 | f | Sb | Riviera 2 1/2 Wo | o.Sb, o.S.e. |
| 28 | m | S.e. Sb | Meer | o.Sb |
| | | | Urlaub 2 Wo | o.Sb, o.S.e. |

m = männlich  
f = weiblich  

Sb = Sonnenbrand  
S.e. = Summer eruption  
o. = ohne  

## Patentansprüche

1. Pharmazeutisches Mittel für die systemische Prophylaxe von Hautschäden, die durch Licht, insbesondere Sonnenlicht, hervorgerufen werden, dadurch **gekennzeichnet**, daß es als Wirkstoff Nicotinsäureamid in Kombination mit Folsäure im Gewichtsverhältnis 10:1 bis 60:1 sowie übliche Trägerstoffe oder Vehicula enthält.

2. Pharmazeutisches Mittel nach Anspruch 1, dadurch gekennzeichnet, daß es Nicotinsäureamid und Folsäure im Gewichtsverhältnis 30:1 bis 50:1 enthält.

3. Pharmazeutisches Mittel nach Anspruch 2, dadurch gekennzeichnet, daß es Nicotinsäureamid und Folsäure im Gewichtsverhältnis 40:1 enthält.

**4.** Pharmazeutisches Mittel nach einem der Ansprüche 1 bis 3 für die orale oder parenterale Verabreichung.

**5.** Verwendung von Nicotinsäureamid und Folsäure zur Herstellung eines oral oder parenteral verabreichbaren pharmazeutischen Mittels für die systemische Prophylaxe von Hautschäden, die durch Licht, insbesondere Sonnenlicht, hervorgerufen werden.

## Claims

**1.** A pharmaceutical compound for the systemic prophylaxis of skin irritations caused by the action of the light, in particular of the sunlight, <u>characterized in that</u> it comprises nicotinic acid amide in combination with folic acid as an active substance in a weight ratio of 10:1 to 60:1 as well as conventional carrier materials or vehicles.

**2.** A pharmaceutical compound according to claim 1, <u>characterized in that</u> it comprises nicotinic acid amide and folic acid in a weight ratio of 30:1 to 50:1.

**3.** A pharmaceutical compound according to claim 2, <u>characterized in that</u> it comprises nicotinic acid amide and folic acid in a weight ratio of 40:1.

**4.** A pharmaceutical compound according to one of claims 1 to 3 for oral or parenteral administration.

**5.** The use of nicotinic acid amide and folic acid for the preparation of a pharmaceutical compound, to be administered orally or parenterally, for the systemic prophylaxis of skin irritations caused by the action of the light, in particular the sunlight.

## Revendications

**1.** Agent pharmaceutique pour la prophylaxie systémique des irritations de la peau provoquées par la lumière, en particulier par le soleil, <u>caractérisé en ce qu</u>'il contient, comme agent, de la nicotinamide en combinaison avec de l'acide folique dans un rapport du poids de 10:1 à 60:1 ainsi que des porteurs habituels ou des véhicules.

**2.** Agent pharmaceutique selon la revendication 1, <u>caractérisé en ce qu</u>'il contient de la nicotinamide et de l'acide folique dans un rapport du poids de 30:1 à 50:1.

**3.** Agent pharmaceutique selon la revendication 2, <u>caractérisé en ce qu</u>'il contient de la nicotinamide et de l'acide folique dans un rapport du poids de 40:1.

**4.** Agent pharmaceutique selon l'une quelconque des revendications 1 à 3 pour l'administration orale ou parentérale.

**5.** L'emploi de nicotinamide et d'acide folique pour la préparation d'un agent pharmaceutique, à administrer de façon orale ou parentérale, pour la prophylaxie systémique des irritations de la peau provoquées par la lumière, en particulier par le soleil.